# EUROPEAN PATENT APPLICATION

(11) **EP 4 678 765 A1**
(43) Date of publication of application: **14.01.2026**
(21) Application number: 24187577.2
(22) Date of filing: 10.07.2024
(51) Int. Cl.: C12Q 1/6874, C12Q 1/6886

(54) **A BIOMARKER PANEL AND METHOD(S) FOR MONITORING OF HEAD AND NECK CANCER**

(71) Applicant: Stichting Amsterdam UMC, 1081 HV Amsterdam (NL)
(72) Inventor: BRAKENHOFF, Rudolf, 1081 HV Amsterdam (NL); POELL, Jos, 1081 HV Amsterdam (NL); BRINK, Arjen, 1081 HV Amsterdam (NL)
(74) Representative: de Pauw, Elmar Sebastian David

(57) **Abstract**

The present disclosure provides a method and a biomarker panel for early detection, monitoring and prognosis of head and neck cancer and its recurrence. Particularly, the method(s) and biomarker panel rely on the enrichment and sequencing of DNA isolated from a biological sample to identify the presence of and/or measure the level of marker(s) selected from a group comprising whole genome of HPV16, E7 sequence(s) of one of more of HPV18, HPV31, HPV33, HPV35, HPV39, HPV45, HPV51, HPV52, HPV56, HPV58, HPV59, HPV68, HPV73 and HPV82, human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63, and combination(s) thereof.

## Description

### TECHNICAL FIELD

The present disclosure relates generally to a genetic analysis for the efficient detection, monitoring and prognosis of head and neck cancer.

### BACKGROUND

Head and neck squamous cell carcinoma (HNSCC) is the sixth most common cancer worldwide and accounts for approximately 5% of all malignancies. It encompasses a heterogeneous group of tumors that arise from the mucosal lining of the upper aerodigestive tract, including the oral cavity, oropharynx, pharynx, and larynx. HNSCC is often diagnosed at an advanced stage, resulting in poor outcomes and high mortality rates. The treatment of HNSCC typically involves a multimodal approach, including surgery, radiotherapy, and concomitant chemoradiotherapy, either alone or in combination.

Several risk factors have been implicated in the development of HNSCC, including tobacco and alcohol consumption, betel nut chewing, and human papillomavirus (HPV) infection. HPV-positive tumors that typically occur in the oropharynx (OPSCC), are considered a distinct clinical entity with unique clinical and molecular characteristics. These tumors are generally treated with definitive radiotherapy or chemoradiotherapy, depending on the disease stage. HPV-positive OPSCC accounts for a significant proportion of all HNSCC cases in Western countries, and the incidence of HPV-positive OPSCC has been rising steadily in recent years. The increasing incidence of HPV-positive HNSCC has important clinical implications, as HPV-positive tumors have been associated with improved treatment response and overall survival when compared to HPV-negative tumors. Nonetheless, two major problems are encountered in the clinical management of patients with HPV-positive OPSCC.

Current disease monitoring after treatment according to WHO guidelines consists of treatment response evaluation by MRI and/or PET-CT three months after treatment completion, and clinical follow-up visits every 6-8 weeks in the first year, every 8-10 weeks in the second year and once every half year in years 3-5. When imaging for response evaluation is performed, residual disease is detected in 10-20%. Residual disease may be detected either at the local site or in the neck, requiring subsequent diagnosis by examination under general anesthesia with biopsies for local recurrence or ultrasound-guided guided fine needle aspiration cytology for diagnosis of the neck, respectively. As samples are taken from heavily treated tissue areas, pathological examination is often not conclusive, leading to potentially unnecessary treatments. On the other hand, recurrences of HPV-positive HNSCC tumors still occur in 15-25% of cases, and form a major challenge for both patients and clinicians, necessitating an adequate clinical follow-up management as watchful surveillance is not without risk. Hence, current monitoring methods have limited sensitivity and specificity, leading to delayed detection of recurrences but also incorrect diagnosis and unnecessary treatments of residual disease with non-vital tumor in the resection specimen. These findings highlight the need for more effective surveillance strategies for HPV-positive HNC patients to test for residual disease and diagnose recurrent OPSCC as early as possible.

Plasma circulating tumor DNA (ctDNA) is a potential biomarker of cancer diagnosis and disease monitoring. Specifically in the context of HPV-positive HNSCC tumors, circulating tumor human papillomavirus (ctHPV) DNA has emerged as a promising biomarker for the detection of HPV-positive OPSCC. CtHPV DNA can be detected in the blood, saliva and oral rinse of HPV-positive HNSCC patients and has been shown to be a sensitive and specific marker for disease recurrence. Different methods for detection have been used, including quantitative PCR, digital droplet PCR (ddPCR) and low coverage whole genome sequencing (IcWGS).

While IcWGS has demonstrated considerable sensitivity to detect ctHPV DNA, there is a need in the art for further improving the sensitivity without reducing specificity.

Given the increasing incidence of HNSCC, the tendency for its recurrence and the complex treatment modules as described above which sometimes may be unnecessary, it is pertinent to detect the disease with the best possible sensitivity and specificity at an early stage to ensure that the quality of life of patients is not compromised. Another major problem in the clinical management of head and neck cancer is the late diagnosis of recurrent disease. When detected early, recurrent tumors can be surgically salvaged and cured. Standard diagnosis for recurrent disease is examination under general anesthesia, but this is obviously unsuited for screening, and only applied on clinical indications. A diagnostic modality that would allow early detection of recurrent disease would form a great development in the management of head and neck cancer. The present disclosure addresses the said need in the art.

### BRIEF DESCRIPTION OF THE FIGURES

In order that the disclosure may be readily understood and put into practical effect, reference will now be made to exemplary embodiments as illustrated with reference to the accompanying figures. The figures together with detailed description below, are incorporated in and form part of the specification, and serve to further illustrate the embodiments and explain various principles and advantages, where:

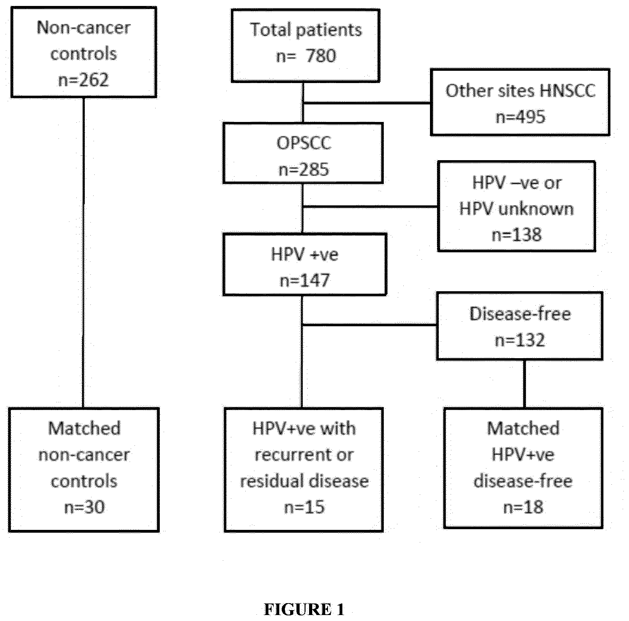

**FIGURE 1** depicts study design of the present disclosure: Patients from two studies with plasma biobanking were combined and samples of patients with HPV-positive OPSCC and non-cancer controls (spouses and caregivers), selected for baseline comparisons. From the patient cohort studied longitudinally, six patients showed residual disease, nine recurrent disease, and 18 were disease-free.

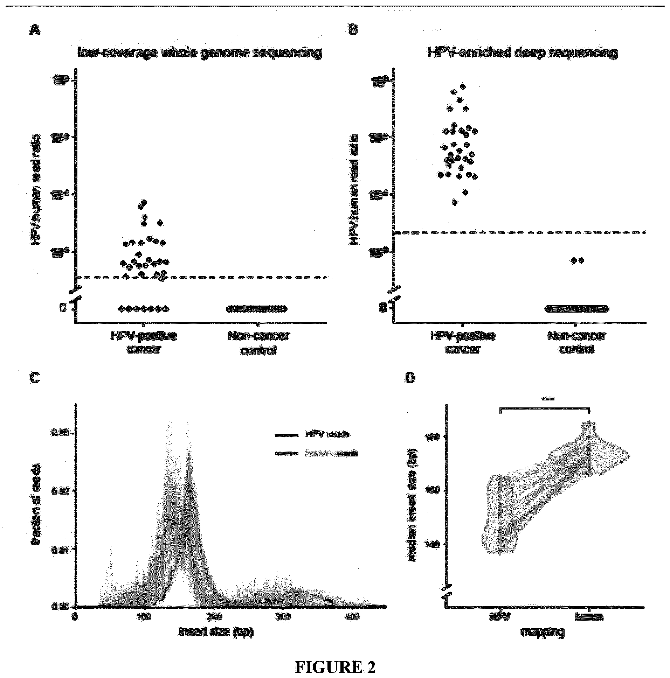

**FIGURE 2** depicts comparison of ctHPV-DNA sequencing: Samples of OPSCC patients were analyzed by low coverage whole genome sequencing (lcWGS: left panel A) and target enrichment DNA sequencing (right panel B). On the Y-axis the number of HPV reads per number of human genome reads. Cut-off levels are indicate with a horizontal bar. In C) the fragment length distributions of HPV (red) and human (blue) genome reads is depicted. In D) the paired median HPV- and human-mapping fragment lengths are compared. The median fragment length of HPV-mapping reads is significantly shorter than that of human-mapping reads (P = 3.0 * 10⁻¹⁴, paired t-test).

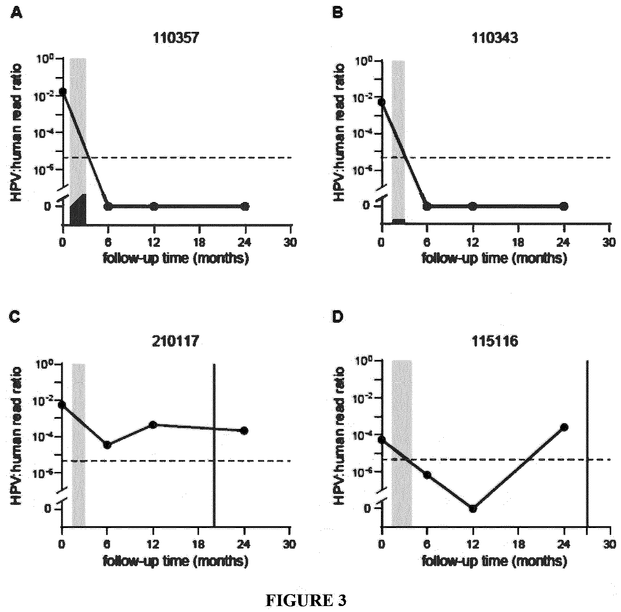

**FIGURE 3** depicts longitudinal disease monitoring in OPSCC patients: Plasma samples taken at baseline, 6, 12 and 24 months were collected and analyzed by target-enrichment sequencing. Plots display the follow-up time in months on the X-axis and HPV:human read ratio on the Y-axis. Green columns represent the treatment period, and a vertical orange line indicates the event: diagnosis of either residual or recurrent disease. The horizontal dashed line indicates the cut-off for ctHPV-DNA positivity. A) and B) display two examples of patients who remained disease-free; C) and D) display two examples of patients who developed recurrent disease.

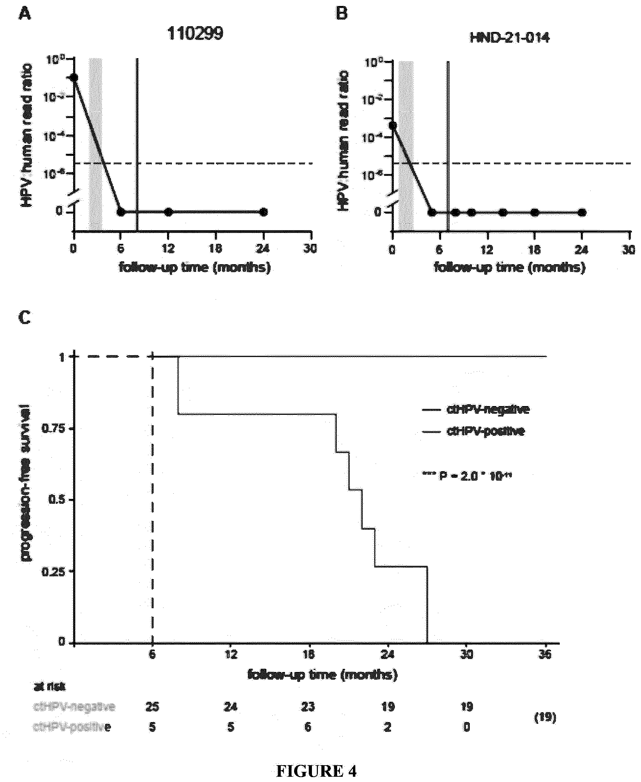

**FIGURE 4** depicts longitudinal disease monitoring in OPSCC patients with residual disease: Plasma samples taken at baseline, 6, 12 and 24 months were collected and analyzed by target-enrichment sequencing. Plots display the follow-up time in months on the X-axis and HPV:human read ratio on the Y-axis. Green columns represent the treatment period, and a vertical orange line indicates the event: diagnosis of either residual or recurrent disease. The horizontal dashed line indicates the cut-off for ctHPV-DNA positivity. A) and B) display two examples of patients with residual disease in the neck and were treated by neck dissection. Non-vital tumor was diagnosed by histopathological examination of the surgical specimen. C) shows disease-free survival of patients of patients stratified by ctHPV-DNA status (as a time-dependent covariate). Time is measured from the first valid measurement after completion of treatment, which was at 6 months for most patients (dashed vertical line). Non-vital tumor residue was not considered an event in this analysis. The number at risk corresponds with ctHPV-DNA status at the specified follow-up time, except at 36 months, when it indicates number of patients still in follow-up. Patients with a ctHPV-DNA positive plasma sample were at significantly higher hazard of recurrence (P = 2.0 * 10⁻¹¹, logrank test).

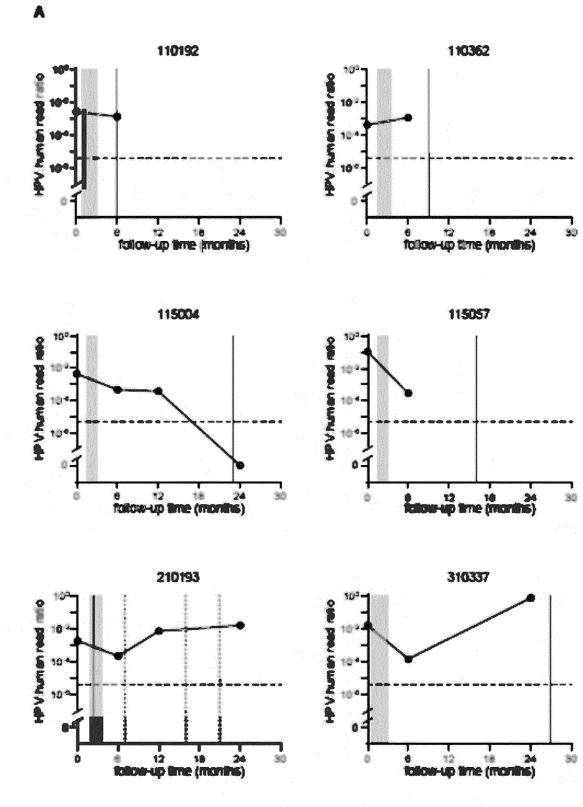

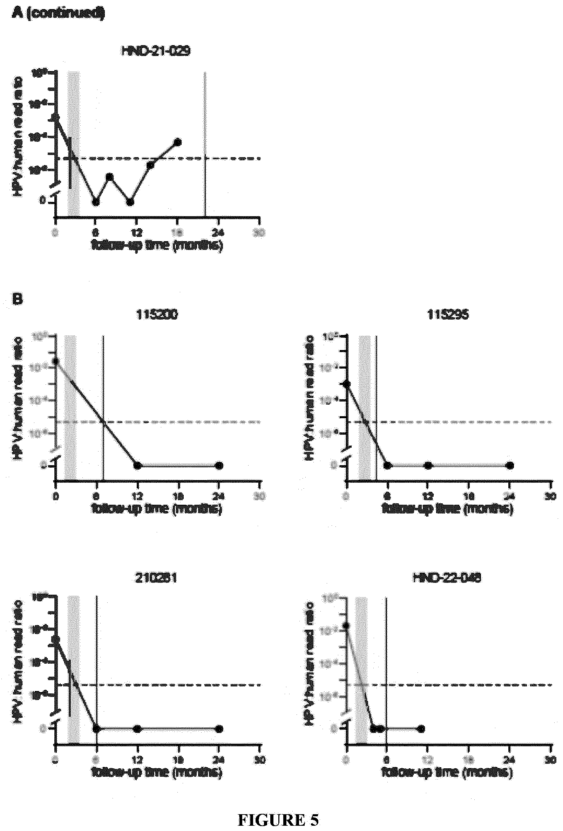

**FIGURE 5** depicts plasma samples taken at baseline, 6, 12 and 24 months were collected and analyzed by target-enrichment sequencing. Plots display the follow-up time in months on the X-axis and HPV:human read ratio on the Y-axis. Green columns represent the treatment period, and a vertical orange line indicates the event: diagnosis of either residual or recurrent disease. Vertical orange dashed line indicates extra events such as distant metastasis. The horizontal dashed line indicates the cut-off for ctHPV-DNA positivity. In A), plots of patients who experienced disease recurrence. In B), plots of patients with residual disease consisting of non-vital tumor cells. Note that plots of patients included in Figures 3 and 4 are not displayed.

### SUMMARY OF THE INVENTION

Addressing the aforesaid need in the art, the present disclosure provides a method for detecting and/or measuring expression of head and neck cancer marker(s) in a biological sample comprising:
a) Isolating DNA from the biological sample; and
b) Performing target-enrichment sequencing analysis on the isolated DNA for confirming presence or absence of marker(s) selected from a group comprising whole genome of HPV16, E7 sequence(s) of one or more of HPV18, HPV31, HPV33, HPV35, HPV39, HPV45, HPV51, HPV52, HPV56, HPV58, HPV59, HPV68, HPV73 and HPV82, and combination(s) thereof.

In some embodiments, the marker(s) may further comprise one or more human genes frequently mutated in head and neck cancer. In some embodiments, the human genes are selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63 or combinations thereof.

In some embodiments, the above method characterized by at least about _% specificity and at least about __% selectivity.

In some embodiments, the method further comprises aligning reads of the HPV and/or human marker(s) to a human genome reference sequence.

Further provided herein is a method of diagnosing head and neck cancer in a subject, comprising:
a) Isolating DNA from a biological sample obtained from a subject; and
b) Performing target-enrichment sequencing analysis on the isolated DNA for confirming presence or absence of marker(s) selected from a group comprising
   ∘ whole genome of HPV16,
   ∘ E7 sequence(s) of one of more of HPV18, HPV31, HPV33, HPV35, HPV39, HPV45, HPV51, HPV52, HPV56, HPV58, HPV59, HPV68, HPV73 and HPV82,
   ∘ human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
   and combination(s) thereof.

In some embodiments, in the above method, the head and neck cancer is recurrent head and neck cancer; and/or the subject is one that has previously received treatment for and/or has previously recovered from head and neck cancer.

In some embodiments, about 3 to about 5 HPV reads per million human genome reads in the target-enrichment sequencing analysis is determined as a cut-off limit for confirming diagnosis of head and neck cancer in the subject.

In some embodiments, about 3 to about 5 HPV reads per million human genome reads in the target-enrichment sequencing analysis is determined as a cut-off limit for confirming diagnosis of recurrent head and neck cancer in the subject.

In some embodiments, provided herein is a method of prognosis of head and neck cancer, comprising:
a) isolating DNA from a biological sample obtained from a subject; and
b) performing target-enrichment sequencing analysis on the isolated DNA for confirming presence or absence of marker(s) selected from a group comprising
   ∘ whole genome of HPV16,
   ∘ E7 sequence(s) of one or more of HPV18, HPV31, HPV33, HPV35, HPV39, HPV45, HPV51, HPV52, HPV56, HPV58, HPV59, HPV68, HPV73 and HPV82,
   ∘ human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
   and combination(s) thereof.

In some embodiments, the prognosis may be used as basis for determining further course of treatment, if required, for head and neck cancer or recurrent head and neck cancer.

Also provided herein is a biomarker enrichment panel comprising probes specific for capture hybridization of biomaker(s) selected from a group comprising
∘ whole genome of HPV16,
∘ E7 sequence(s) of one or more of HPV18, HPV31, HPV33, HPV35, HPV39, HPV45, HPV51, HPV52, HPV56, HPV58, HPV59, HPV68, HPV73 and HPV82,
∘ human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
and combination(s) thereof for detection of head and neck cancer(s).

In some embodiments, the Human:HPV enrichment probes are pooled at a ratio of about 2:1 in the biomarker panel.

The present disclosure further provides use of maker(s) selected from a group compri sing
whole genome of HPV16,
E7 sequence(s) of one or more of HPV18, HPV31, HPV33, HPV35, HPV39, HPV45, HPV51, HPV52, HPV56, HPV58, HPV59, HPV68, HPV73 and HPV82, human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
and combination(s) thereof for detection of head and neck cancer(s).

In some embodiments, the head and neck cancer is a recurrent head and neck cancer.

Further envisaged herein is use of target enrichment analysis for detecting and/or measuring expression of head and neck cancer marker(s) selected from a group comprising
∘ whole genome of HPV16,
∘ E7 sequence(s) of one or more of HPV18, HPV31, HPV33, HPV35, HPV39, HPV45, HPV51, HPV52, HPV56, HPV58, HPV59, HPV68, HPV73 and HPV82,
∘ human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
and combination(s) thereof
in a biological sample for detection of head and neck cancer(s).

### DETAILED DESCRIPTION

### Definitions

As used herein, the term "head and neck cancer" or Head and neck squamous cell carcinoma or HNSCC is a general term encompassing multiple cancers that can develop in the head and neck region. The said term includes under its scope oropharyngeal cancer which is cancer in the oropharynx which is the middle section of the throat (pharynx) that makes saliva, keeps the mouth and throat moist and helps in digestion of food.

The term "Human Papillomavirus" has been used interchangeably with "HPV". Human papillomavirus (HPV) is the name of a very common group of viruses. HPV is a common virus that can cause certain cancers later in life. The contribution of HPV to HNSCC is common within the tonsillar crypt in the oropharynx.

As used herein, the term "marker" and "biomarker" have been used interchangeably refer to a biological molecule or differentially expressed gene found in blood, other body fluids, or tissues that is a sign of a normal or abnormal process, or of a condition or disease

The term "Circulating tumor DNA" or "ctDNA" refers to DNA that comes from cancerous cells and tumors and is found in the bloodstream. Most DNA is inside a cell's nucleus. As a tumor grows, cells die and are replaced by new ones. The dead cells get broken down and their contents, including DNA, are released into the bloodstream. CtDNA is therefore a type of cell free DNA. Reference in the present disclosure to HPV genes that are detected and quantified in biological samples, therefore, implies reference to HPV ctDNA.

As used herein, the term "subject" is a vertebrate, such as a mammal, such as a human. Mammals include, but are not limited to, humans, livestock, athletic animals, pets and the like.

As used herein, the term "comprising" when placed before the recitation of steps in a method means that the method encompasses one or more steps that are additional to those expressly recited, and that the additional one or more steps may be performed before, between, and/or after the recited steps. For example, a method comprising steps a, b, and c encompasses a method of steps a, b, x, and c, a method of steps a, b, c, and x, as well as a method of steps x, a, b, and c. Furthermore, the term "comprising" when placed before the recitation of steps in a method does not (although it may) require sequential performance of the listed steps, unless the content clearly dictates otherwise. For example, a method comprising steps a, b, and c encompasses, for example, a method of performing steps in the order of steps a, c, and b, the order of steps c, b, and a, and the order of steps c, a, and b, etc.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. The suffix "(s)" at the end of any term in the present disclosure envisages in scope both the singular and plural forms of said term.

As used in this specification and the appended claims, the singular forms "a," "an" and "the" includes both singular and plural references unless the content clearly dictates otherwise. For example, the term "inserted at a position" as used herein in reference to a polypeptide sequence refers to insertion at one or more (such as one, two, three, etc.) amino acid positions in the polypeptide sequence. The use of the expression `at least' or `at least one' suggests the use of one or more elements or ingredients or quantities, as the use may be in the embodiment of the disclosure to achieve one or more of the desired objects or results. As such, the terms "a" (or "an"), "one or more", and "at least one" can be used interchangeably herein.

Numerical ranges stated in the form 'from x to y' include the values mentioned and those values that lie within the range of the respective measurement accuracy as known to the skilled person. If several preferred numerical ranges are stated in this form, of course, all the ranges formed by a combination of the different end points are also included.

The terms "about" or "approximately" as used herein when referring to a measurable value such as a parameter, an amount, a temporal duration, and the like, are meant to encompass variations of and from the specified value, such as variations of +/-10% or less, +/-5% or less, +/-1% or less, and +/-0.1% or less of and from the specified value, insofar such variations are appropriate to perform in the disclosed invention. It is to be understood that the value to which the modifier "about" or "approximately" refers is itself also specifically, and preferably, disclosed.

As used herein, the terms "include" (any form of "include", such as "include"), "have" (and "have"), "comprise" etc. any form of "having", "including" (and any form of "including" such as "including"), "containing", "comprising" or "comprises" are inclusive and will be understood to imply the inclusion of a stated element, integer or step, or group of elements, integers or steps, but not the exclusion of any other element, integer or step, or group of elements, integers or steps

As regards the embodiments characterized in this specification, it is intended that each embodiment be read independently as well as in combination with another embodiment. For example, in case of an embodiment 1 reciting 3 alternatives A, B and C, an embodiment 2 reciting 3 alternatives D, E and F and an embodiment 3 reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

### Disclosure

The present disclosure demonstrates that target-enrichment DNA sequencing of HPV genome(s) or parts thereof allows early and efficient detection of head and neck cancers, especially those recurrent in individuals who have received treatment for the disease. In some embodiments, the present disclosure particularly relies on whole genome of HPV16 as it comprises more than 90% of all infections in OPSCC patients, along with E7 sequences of the other high-risk types. It is a surprising finding that the elevated sensitivity reported in the present disclosure does not come at the cost of specificity.

The methodology reported in the present disclosure further relies on a panel of cancer genes frequently mutated in HNSCC to allow application for HPV-negative tumors. The genomic data provides an opportunity for quality control analyses and also allows quantifications and considerations to determine if a sample was diagnostic or not. When combined with the circulating tumour HPV-DNA (ctHPV-DNA) fraction analysis of samples, this approach provides a tremendous opportunity for diagnostic application.

The results of the studies reported in the present disclosure suggest that target-enrichment deep sequencing of ctHPV-DNA in liquid biopsy plasma samples is a powerful tool for monitoring ctHPV-DNA after treatment for HNSCC. The high sensitivity and specificity of the target enrichment sequencing using a panel including HPV16 and E7 sequences of other high risk HPV types permits early detection of recurrence, which could lead to earlier intervention and improved outcomes for patients.

Said aspects of the present disclosure are explained in further detail in the paragraphs that follow

### A method for detecting and/or measuring expression of head and neck cancer marker(s) in a biological sample

The present disclosure provides a method for detection of marker(s) of head and neck cancer in a biological sample, comprising:
- isolating DNA from the biological sample; and
- performing target-enrichment sequencing analysis on the isolated DNA for confirming presence or absence of one or more of ctHPV-DNA marker(s) selected from a group comprising whole genome of HPV16, HPV18, HPV31, HPV33, HPV35, HPV39, HPV45, HPV51, HPV52, HPV56, HPV58 and HPV59 or any part thereof or combinations thereof.

In some embodiments, the method for detection of marker(s) of head and neck cancer in a biological sample comprises:
- isolating DNA from the biological sample; and
- performing target-enrichment sequencing analysis on the isolated DNA for confirming presence or absence of one or more of ctHPV-DNA marker(s) selected from a group comprising
   ∘ whole genome of HPV16,
   ∘ E7 sequence(s) of one or more of HPV18, HPV31, HPV33, HPV35, HPV39, HPV45, HPV51, HPV52, HPV56, HPV58, and HPV59,
   and combination(s) thereof.

In some embodiments, the method for detection of marker(s) of head and neck cancer in a biological sample comprises:
- isolating DNA from the biological sample; and
- performing target-enrichment sequencing analysis on the isolated DNA for confirming presence or absence of marker(s) selected from a group comprising
   ∘ whole genome of HPV16, and
   ∘ E7 sequence(s) of one or more of HPV18, HPV31, HPV33, HPV35, HPV39, HPV45, HPV51, HPV52, HPV56, HPV58, HPV59, HPV68, HPV73 and HPV82,
   and combination(s) thereof.

In some embodiments, the marker(s) may further comprise one or more human genes frequently mutated in head and neck cancer.

In some embodiments, the human genes are selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63 or combinations thereof.

Accordingly, in some embodiments, provided herein is a method for detection of marker(s) of head and neck cancer in a biological sample, comprising:
- isolating DNA from the biological sample; and
- performing target-enrichment sequencing analysis on the isolated DNA for confirming presence or absence of marker(s) selected from a group comprising
   ∘ whole genome of HPV16,
   ∘ E7 sequence(s) of one or more of HPV18, HPV31, HPV33, HPV35, HPV39, HPV45, HPV51, HPV52, HPV56, HPV58, HPV59, HPV68, HPV73 and HPV82,
   ∘ human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
   and combination(s) thereof.

In a non-limiting embodiment, in the aforesaid method of the present disclosure, the prior to performing the target-enrichment sequencing analysis, the isolated DNA may be used to generate sequencing libraries. The sequencing libraries may be subsequently used for target-enrichment to generate enriched libraries. Further, the enriched libraries may be used for sequencing. The said sequencing, in some embodiments, may yield paired end reads. Without intending to be limited by theory, the generation of sequencing libraries, sequencing libraries and subsequent sequencing together constitute the target-enrichment sequencing analysis. In a non-limiting embodiment, the generation of sequencing libraries, sequencing libraries and subsequent sequencing may be performed by any methodology or protocol known in the art.

In some embodiments, the biological sample is a liquid biopsy sample.

In some embodiments, the liquid biopsy sample is selected from a group comprising blood, plasma and saliva.

In an exemplary embodiment, the liquid biopsy sample is plasma.

In yet another exemplary embodiment, the liquid biopsy sample comprises longitudinal plasma sample(s).

In some embodiments, the aforesaid method of the present disclosure is characterized by at least about 90% specificity and/or at least about 90% selectivity.

In some embodiments, the aforesaid method of the present disclosure is characterized by about 90% to about 100% specificity and/or about 90% to about 100% selectivity.

In some embodiments, the aforesaid method of the present disclosure is characterized by about 90%, about 91%, about 92%, about 93%, about 94%, about 95%, about 96%, about 97%, about 98%, about 99% or about 100% specificity.

In some embodiments, the aforesaid method of the present disclosure is characterized by about 90%, about 91%, about 92%, about 93%, about 94%, about 95%, about 96%, about 97%, about 98%, about 99% or about 100% selectivity.

In an exemplary embodiment, the aforesaid method of the present disclosure is characterized by about 100% specificity and/or about 100% selectivity.

In another exemplary embodiment, the aforesaid method of the present disclosure is characterized by about 100% specificity and about 100% selectivity.

In some embodiments, the aforesaid method of the present disclosure further comprises aligning reads of the HPV and/or human marker(s) to a human genome reference sequence.

In a non-limiting embodiment, the aforesaid method of the present disclosure also allows mapping location of the reads of the HPV along the HPV genome and also allows determining or estimating fragment length distribution. This in turn may find application in facilitating genetic mapping-guided surgery.

### Diagnosis and monitoring of head and neck cancer

In addition to the above, in some embodiments, provided herein is a method of diagnosing head and neck cancer, comprising:
a) isolating DNA from a biological sample obtained from a subject; and
b) performing target-enrichment sequencing analysis on the isolated DNA for confirming presence or absence of marker(s) selected from a group comprising

∘ whole genome of HPV16,
∘ E7 sequence(s) of one or more of HPV18, HPV31, HPV33, HPV35, HPV39, HPV45, HPV51, HPV52, HPV56, HPV58, HPV59, HPV68, HPV73 and HPV82,
∘ human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
and combination(s) thereof.

In some embodiments, the subject may be one who has previously been diagnosed with head and neck cancer and has received treatment for the same.

In some embodiments, the subject may be one who has previously been diagnosed with head and neck cancer, has received treatment for the same and has been declared cancer free.

In some embodiments, the subject may be one who is undergoing periodic monitoring or follow-up checkups after receiving treatment for and/or recovering from head and neck cancer.

Thus, in some embodiments, provided herein is a method of diagnosing recurrent head and neck cancer in a subject, comprising:
c) isolating DNA from a biological sample obtained from the subject; and
d) performing target-enrichment sequencing analysis on the isolated DNA for confirming presence or absence of marker(s) selected from a group comprising
   ∘ whole genome of HPV16,
   ∘ E7 sequence(s) of one or more of HPV18, HPV31, HPV33, HPV35, HPV39, HPV45, HPV51, HPV52, HPV56, HPV58, HPV59, HPV68, HPV73 and HPV82,
   ∘ human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
   and combination(s) thereof.

In some embodiments, the diagnosis for head and neck cancer or recurrent head and neck cancer is confirmed if one or more of the marker(s) are found to meet a predetermined cut-off value.

Accordingly, in some embodiments, provided herein is a method of diagnosing head and neck cancer in a subject, comprising:
a) isolating DNA from a biological sample obtained from the subject; and
b) performing target-enrichment sequencing analysis on the isolated DNA for confirming presence or absence of marker(s) selected from a group comprising
   ∘ whole genome of HPV16,
   ∘ E7 sequence(s) of one or more of HPV18, HPV31, HPV33, HPV35, HPV39, HPV45, HPV51, HPV52, HPV56, HPV58, HPV59, HPV68, HPV73 and HPV82,
   ∘ human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
   and combination(s) thereof,
   wherein the subject is confirmed to be diagnosed with head and neck cancer if one or more of the marker(s) in the biological sample are found to meet a predetermined cut-off value.

In some embodiments, provided herein is a method of diagnosing recurrent head and neck cancer in a subject, comprising:
a) isolating DNA from a biological sample obtained from the subject; and
b) performing target-enrichment sequencing analysis on the isolated DNA for confirming presence or absence of marker(s) selected from a group comprising
   ∘ whole genome of HPV16,
   ∘ E7 sequence(s) of one or more of HPV18, HPV31, HPV33, HPV35, HPV39, HPV45, HPV51, HPV52, HPV56, HPV58, HPV59, HPV68, HPV73 and HPV82,
   ∘ human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
   and combination(s) thereof,
   wherein the subject is confirmed to be diagnosed with recurrent head and neck cancer if one or more of the marker(s) in the biological sample are found to meet a pre-determined cut-off value.

In a non-limiting embodiment, about 3 HPV to about 5 HPV reads per million human genome reads in the target-enrichment sequencing analysis is determined as a cut-off limit for confirming diagnosis of recurrent head and neck cancer in the subject.

In a non-limiting embodiment, about 3 HPV, about 3.5 HPV, about 4HPV, about 4.5 HPV or about 5 HPV reads per million human genome reads in the target-enrichment sequencing analysis is determined as a cut-off limit for confirming diagnosis of recurrent head and neck cancer in the subject.

In an exemplary embodiment, about 4.7 HPV reads per million human genome reads in the target-enrichment sequencing analysis is determined as a cut-off limit for confirming diagnosis of recurrent head and neck cancer in the subject.

In a non-limiting embodiment, in the aforesaid method of the present disclosure, the prior to performing the target-enrichment sequencing analysis, the isolated DNA may be used to generate sequencing libraries. The sequencing libraries may be subsequently used for target-enrichment to generate enriched libraries. Further, the enriched libraries may be used for sequencing. The said sequencing, in some embodiments, may yield paired end reads. Without intending to be limited by theory, the generation of sequencing libraries, sequencing libraries and subsequent sequencing together constitute the target-enrichment sequencing analysis. In a non-limiting embodiment, the generation of sequencing libraries, sequencing libraries and subsequent sequencing may be performed by any methodology or protocol known in the art.

In some embodiments, the biological sample is a liquid biopsy sample.

In some embodiments, the liquid biopsy sample is selected from a group comprising blood, plasma and saliva.

In an exemplary embodiment, the liquid biopsy sample is plasma.

In yet another exemplary embodiment, the liquid biopsy sample comprises longitudinal plasma sample(s). In the setting of longitudinal monitoring the presence of ctHPV-DNA in plasma often precedes a recurrence when tested. Importantly, ctHPV-DNA positivity in plasma indicates a highly elevated hazard, since ctHPV-DNA is only detected longitudinally in the recurrence-free group in one sample during post-treatment follow-up, as explained in the working examples.

In some embodiments, a salient feature of the present disclosure is the finding that ctHPV-DNA negativity in a biological sample predicts non-vital status of residual tumor mass after treatment.

Thus, in some embodiments, envisaged herein is a method of predicting and/or confirming non-vital status of residual tumor mass in a subject, comprising:
a) isolating DNA from a biological sample obtained from the subject; and
b) performing target-enrichment sequencing analysis on the isolated DNA for confirming presence or absence of marker(s) selected from a group comprising
   ∘ whole genome of HPV16,
   ∘ E7 sequence(s) of one or more of HPV18, HPV31, HPV33, HPV35, HPV39, HPV45, HPV51, HPV52, HPV56, HPV58, HPV59, HPV68, HPV73 and HPV82,
   ∘ human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
   and combination(s) thereof to confirm negativity of the marker(s) and therefore non-vital status of the residual tumor mass.

For the purposes of clarity, `negativity of the marker(s)' in the above embodiment may refer to complete absence of the marker(s) or presence of the marker(s) below the predetermined cut-off limit.

In some embodiments, the aforesaid method of the present disclosure is characterized by at least about 90% specificity and/or at least about 90% selectivity.

In some embodiments, the aforesaid method of the present disclosure is characterized by about 90% to about 100% specificity and/or about 90% to about 100% selectivity.

In some embodiments, the aforesaid method of the present disclosure is characterized by about 100% specificity and/or about 100% selectivity.

In some embodiments, the aforesaid method of the present disclosure is characterized by about 100% specificity and about 100% selectivity.

### Prognosis of head and neck cancer

In addition to the above, in some embodiments, provided herein is a method of prognosis of head and neck cancer, comprising:
c) isolating DNA from a biological sample obtained from a subject; and
d) performing target-enrichment sequencing analysis on the isolated DNA for confirming presence or absence of marker(s) selected from a group comprising
   ∘ whole genome of HPV16,
   ∘ E7 sequence(s) of one or more of HPV18, HPV31, HPV33, HPV35, HPV39, HPV45, HPV51, HPV52, HPV56, HPV58, HPV59, HPV68, HPV73 and HPV82,
   ∘ human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
   and combination(s) thereof.

In some embodiments, the subject may be one who has previously been diagnosed with head and neck cancer and has received treatment for the same.

In some embodiments, the subject may be one who has previously been diagnosed with head and neck cancer, has received treatment for the same and has been declared cancer free.

In some embodiments, the subject may be one who is undergoing periodic monitoring or follow-up checkups after receiving treatment for and/or recovering from head and neck cancer.

In some embodiments, the prognosis may be used as basis for determining further course of treatment, if required, for head and neck cancer or recurrent head and neck cancer.

### Biomarker enrichment panel

Further provided in the present disclosure is a biomarker enrichment panel comprising probes specific for capture hybridization of maker(s) selected from a group comprising
∘ whole genome of HPV16,
∘ E7 sequence(s) of one or more of HPV18, HPV31, HPV33, HPV35, HPV39, HPV45, HPV51, HPV52, HPV56, HPV58, HPV59, HPV68, HPV73 and HPV82,
∘ human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
and combination(s) thereof for detection of head and neck cancer(s).

In a non-limiting embodiments, the Human:HPV enrichment probes are pooled at a ratio ranging of about 2:1.

In some embodiments, design of the probes specific for capture hybridization of maker(s) would be well within the purview of knowledge of a skilled artisan in view of the sequences of the marker(s) *per se* being known in the art.

In some embodiments, the present disclosure further envisages a system for target-enrichment sequencing analysis of a sample for diagnosis of head and neck cancer or recurrent head and neck cancer, comprising the biomarker enrichment panel as described above.

### Application(s) of the biomarker(s), the methodology employed in the present disclosure and the biomarker panel as described above

Further envisaged herein is use of maker(s) selected from a group comprising whole genome of HPV16,
E7 sequence(s) of one or more of HPV18, HPV31, HPV33, HPV35, HPV39, HPV45, HPV51, HPV52, HPV56, HPV58, HPV59, HPV68, HPV73 and HPV82, human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63
and combination(s) thereof for detection of head and neck cancer(s).

In some embodiments, the head and neck cancer is a recurrent head and neck cancer.

Also provided herein is use of target enrichment analysis for detecting and/or measuring expression of head and neck cancer marker(s) selected from a group comprising
∘ whole genome of HPV16,
∘ E7 sequence(s) of one or more of HPV18, HPV31, HPV33, HPV35, HPV39, HPV45, HPV51, HPV52, HPV56, HPV58, HPV59, HPV68, HPV73 and HPV82,
∘ human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
and combination(s) thereof
in a biological sample for detection of head and neck cancer(s).

In some embodiments, further provided herein is use of the biomarker enrichment panel as described above for enrichment sequencing analysis of a sample for facilitating diagnosis of head and neck cancer or recurrent head and neck cancer in a subject.

In some embodiments, measurements of ctHPV-DNA by the method as described above may also be used to prevent overtreatment. The method(s) of the present disclosure are likely to ensure residual and recurrent disease is detected at least in all cases on time or earlier than by current standard practice, with limited or none false positive findings. This might prevent unnecessary imaging as well as examinations under general anesthesia and futile neck dissections.

The results of the studies reported in the present disclosure suggest that target-enrichment deep sequencing of HPV DNA in plasma samples is a powerful tool for monitoring ctHPV-DNA after treatment for HNSCC. The high sensitivity and specificity of our target enrichment sequencing using a panel including HPV16 and E7 sequences of other high risk HPV types permits early detection of recurrence, which could lead to earlier intervention and improved outcomes for patients. Based on the data presented, a complete substitution of the current follow-up policy by liquid biopsy might become an option in the foreseeable future.

The subject matter of embodiments of the present disclosure is described here with specificity to meet statutory requirements, but this description is not necessarily intended to limit the scope of the disclosure. The disclosed subject matter may be embodied in other ways, may include different elements or steps, and may be used in conjunction with other existing or future technologies. This description should not be interpreted as implying any particular order or arrangement among or between various steps or elements except when the order of individual steps or arrangement of elements is explicitly described.

### EXAMPLES

### Materials and methods - patients, sample collection, DNA isolation and sequencing

### Patients

Patient data and samples were obtained from HNSCC patients and caregivers/spouses as non-cancer controls participating in the prospective NETherlands Quality of Life and Biomedical Cohort study in HNC cancer (NET-QUBIC). Recruitment of the 739 HNSCC patients and 262 caregivers/spouses took place at 7 different head and neck cancer centers in the Netherlands between 2014 and 2018. Inclusion criteria were age above 17, diagnosis of oral, oropharyngeal, hypopharyngeal, or laryngeal HNSCC, and treatment with curative intent (all treatment modalities). Exclusion criteria were recurrent or residual disease at baseline, cognitive impairments, other tumors than HNSCC, and insufficient knowledge of Dutch language. All patients and non-cancer controls signed written informed consent before participation. The study was approved by the Institutional Review Board of Amsterdam UMC location VUmc and all participating centers. Patient data includes baseline characteristics with demographic factors, clinical factors, treatment, comorbidities and disease outcome parameters. Plasma and oral rinse samples were available at different time points: at baseline, 6 months, 12 months and 24 months after histological diagnosis. In total 262 patients were diagnosed with oropharyngeal squamous cell carcinoma (OPSCC) of which 155 displayed a p16-positive immunostaining. Patients were included only when the tumor was tested positive for HPV-DNA or baseline plasma samples were positive (n=4).

For the case-control post-treatment monitoring study, 33 patients with HPV-positive OPSCC were selected of whom 18 patients remained disease-free for 24 months, six patients were suspected for residual disease in the neck at response monitoring imaging and were treated by neck dissection, and all 9 who developed recurrent disease during follow-up. In total 30 caregivers (usually spouses) served as non-cancer controls. To overcome selection bias, OPSCC patients and non-cancer controls were matched by propensity scores for age, smoking (current, former, never) and alcohol (ever, never). The patients with HPV-positive OPSCC who remained disease-free were matched to the group of patients with residual/recurrent disease for treatment and stage.

### Blood samples

Blood was collected in 4 x 6 ml EDTA blood vials and plasma separated by centrifugation at about 500g for about 20 min. Plasma was transferred to microcentrifuge vials and centrifuged at 21,000g for 10 minutes and pipetted to a new microcentrifuge vial. Alternatively, the blood was centrifuged for about 20 minutes at about 120g, plasma transferred to a new vial and centrifuged for about 360 g for about 20 minutes, again transferred and spun for about 10 minutes at about 21,000g and transferred to a new vial. All centrifugation steps were done at room temperature. Plasma was always isolated and frozen within 3 hours after blood withdrawal. In total about 3-4 mL plasma was thawed for each available time point of all patients. DNA isolation was performed using the QIAamp circulating nucleic acid kit for cell-free DNA (Qiagen) following the protocol of the manufacturer and eluted with 60 µl elution buffer. DNA concentration was measured and quality control performed by loading about 2 µl on a cfDNA ScreenTape (Agilent TapeStation).

### Oral rinses

Patients rinsed their oral cavity with about 10 ml of about 0.9% sterile saline from a small plastic cup and collected the rinse in the same cup. The rinses were aliquoted in 2ml vials and frozen. For DNA isolation, the vials were thawed and DNA isolation was performed using the QIAamp circulating nucleic acid kit for cell-free DNA following the protocol of the manufacturer, with extra proteinase K during the lysis step and eluted with about 60 µl elution buffer. DNA concentration was measured and quality control performed using the Qubit dsDNA High Sensitivity kit (Thermo Fisher Scientific).

### DNA Isolation

DNA was isolated from about 3-4 ml of plasma, eluted and was loaded on a Tapestation cfDNA Screentape to determine the % of cfDNA and the concentration of baseline plasma samples. Generally, the median % of cfDNA was found to be about 81%. The mean plasma DNA yield was about 5.3 ng/ml for the OPSCC patients (range 0.15-14.4) and about 5.7 ng/ml for the caregivers (range 2.1-21.6), these groups were not significantly different (p=0.9 by Students T-test).

### DNA Sequencing

In total about 50 µl of DNA eluate was used for sequencing. The sequencing libraries were generated using the KAPA HyperPrep Kit (KAPA Biosystems) including the use of unique molecular indexes (UMIs) and dual sample indexes, following the KAPA_HyperCap_cfDNA_Workflow_v1.1 of the vendor.

For low coverage whole genome sequencing analyses, libraries of 24 samples were equimolarly pooled and sequenced for 150 bp paired-end reads on an Illumina NovaSeq 6000. The generated sequencing libraries were used for target-enrichment using KAPA HyperCap protocol (Roche). The capture panel consisted of 32 human genes (including 29 frequently mutated head and neck cancer genes), the full genome of HP16, and the E7 sequences of all other 14 high risk HPV types (Table 1). Human:HPV enrichment probes were pooled at a ratio of 2:1 in the final panel. Target enrichment was performed as described by the vendor. Equimolar pooling of 24 samples in a combined amount of 1-2 µg DNA was attempted. The enriched libraries were sequenced on an Illumina NovaSeq 6000 for 150 bp paired-end reads.

**Table 1:**

| **Human/HPV** | **Biomarkers** |
|---|---|
| **Human Genes** | AJUBA |
| | ASXL1 |
| | CASP8 |
| | CDKN2A |
| | CUL3 |
| | DDX3X |
| | EPHA2 |
| | FAT1 |
| | FBXW7 |
| | FGFR3 |
| | HRAS |
| | KDM6A |
| | KEAP1 |
| | KMT2D |
| | KRAS |
| | NFE2L2 |
| | NOTCH1 |
| | NOTCH2 |
| | NSD1 |
| | PIK3CA |
| | PTEN |
| | RB1 |
| | RHOA |
| | TERT promotor |
| | TGFBR2 |
| | TP53 |
| | TP53 5'UTR |
| | TP63 |
| | ZNF750 |
| | |
| **HPV 16 (whole genome)** | HPV 16 |
| | |
| **HPV18** | E7 sequence |
| **HPV31** | E7 sequence |
| **HPV33** | E7 sequence |
| **HPV35** | E7 sequence |
| **HPV39** | E7 sequence |
| **HPV45** | E7 sequence |
| **HPV51** | E7 sequence |
| **HPV52** | E7 sequence |
| **HPV56** | E7 sequence |
| **HPV58** | E7 sequence |
| **HPV59** | E7 sequence |
| **HPV68** | E7 sequence |
| **HPV73** | E7 sequence |
| **HPV82** | E7 sequence |

### Analyses

Reads were mapped using BWA mem (0.7.17) to the human genome hg19 extended with all 15 high-risk HPV genomes.

For low coverage whole genome sequencing (1cWGS) data duplicate reads were removed using GATK (4.3.0.0) MarkDuplicates. For target-enriched data duplicate reads were removed using the UMI information with fgbio (2.1.1) GroupReadsByUmi, CallMolecularConsensusReads and FilterConsensusReads.

Unique HPV reads and human genome reads were separately counted using samtools (1.16.1) for lcWGS data. For the target-enriched data on-target HPV and human reads were extracted using bedtools (2.29.2). For lcWGS the number of HPV reads per million human genome reads was taken as readout with a cut-off of 0.125 for positive and negative.

For target-enrichment sequencing, the absolute number of HPV reads and the number of HPV reads per million human genome reads were calculated as final readout.

In addition, variants were called by GATK (4.4.0.0) Mutect2 and VarScan 2 (2.4.4) for SNP analysis. The germline SNPs were correlated in a correlation plot comparison to confirm sample identities. Intrapatient SNP variant allele frequencies always had a correlation coefficent of > 0.893, while interpatient SNP variant allele frequencies always had a correlation coefficient < 0.799 In addition HPV16 variants were manually checked, and HPV genome coverage profiles examined. All these data were used to corroborate sample identity.

Total human genome reads were used to calculate whether a sample was diagnostic.

### Example 1: Comparison of ctHPV-DNA analysis using IcWGS and target-enrichment sequencing

Using low coverage whole genome sequencing (lcWGS), ctHPV-DNA at baseline was detected in the plasma of 25 out of 33 patients with HPV-positive OPSCCs, and in none of the 21 non-cancer controls (Figure 2A). This thus showed a sensitivity of about 75.8% and a specificity of about 100%. The quality control parameters showed a sufficient concentration of circulating cell-free DNA (cfDNA) for library preparation and sufficient human genome reads in all patients (Table 2).

**Table 2:**

| **Sample** | **Group** | **Human reads** | **HPV16 reads** | **HPV16:Human (x 1E+06)** |
|---|---|---|---|---|
| 110192-T0-Plasma | Relapse | 11646514 | 2 | 0.17 |
| 110299-T0-Plasma | Relapse | 11729099 | 114 | 9.72 |
| 110362-T0-Plasma | Relapse | 12139124 | 2 | 0.16 |
| 115116-T0-Plasma | Relapse | 12796836 | 0 | 0 |
| 210117-T0-Plasma | Relapse | 11630924 | 6 | 0.52 |
| 210193-T0-Plasma | Relapse | 11870330 | 4 | 0.34 |
| 210281-T0-Plasma | Relapse | 12727793 | 10 | 0.79 |
| 310337-T0-Plasma | Relapse | 14227277 | 32 | 2.25 |
| 21-014-T0-Plasma | Relapse | 15750466 | 6 | 0.38 |
| 21-029-T0-Plasma | Relapse | 14351787 | 2 | 0.14 |
| 22-048-T0-Plasma | Relapse | 20195732 | 0 | 0 |
| 115004-T0-Plasma | Relapse | 10236990 | 4 | 0.39 |
| 115057-T0-Plasma | Relapse | 12132695 | 123 | 10.14 |
| 115200-T0-Plasma | Relapse | 15838637 | 44 | 2.78 |
| 115295-T0-Plasma | Relapse | 13803652 | 6 | 0.43 |
| 21-013-T0-Plasma | Disease free follow-up | 13684683 | 28 | 2.05 |
| 21-040-T0-Plasma | Disease free follow-up | 39392393 | 0 | 0 |
| 210199-T0-Plasma | Disease free follow-up | 9569555 | 0 | 0 |
| 110187-T0-Plasma | Disease free follow-up | 13358630 | 6 | 0.45 |
| 110240-T0-Plasma | Disease free follow-up | 12014794 | 24 | 2.00 |
| 110251-T0-Plasma | Disease free follow-up | 12298290 | 4 | 0.33 |
| 110343-T0-Plasma | Disease free follow-up | 13175957 | 6 | 0.46 |
| 110357-T0-Plasma | Disease free follow-up | 12247367 | 22 | 1.80 |
| 110383-T0-Plasma | Disease free follow-up | 16965031 | 2 | 0.12 |
| 115168-T0-Plasma | Disease free follow-up | 14248937 | 0 | 0 |
| 115315-T0-Plasma | Disease free follow-up | 10847212 | 2 | 0.18 |
| 115376-T0-Plasma | Disease free follow-up | 14079868 | 28 | 1.99 |
| 116037-T0-Plasma | Disease free follow-up | 13703923 | 0 | 0 |
| 210005-T0-Plasma | Disease free follow-up | 13663500 | 4 | 0.29 |
| 210200-T0-Plasma | Disease free follow-up | 13769594 | 511 | 37.11 |
| 210348-T0-Plasma | Disease free follow-up | 13439394 | 711 | 52.90 |
| 210457-T0-Plasma | Disease free follow-up | 11844521 | 0 | 0 |
| 410511-T0-Plasma | Disease free follow-up | 14084797 | 232 | 16.47 |
| 120186-T0-Plasma | Non-cancer control | 11897011 | 0 | 0 |
| 120220-T0-Plasma | Non-cancer control | 12573362 | 0 | 0 |
| 120265-T0-Plasma | Non-cancer control | 12665158 | 0 | 0 |
| 120272-T0-Plasma | Non-cancer control | 13928625 | 0 | 0 |
| 125197-T0-Plasma | Non-cancer control | 17061050 | 0 | 0 |
| 125245-T0-Plasma | Non-cancer control | 15263020 | 0 | 0 |
| 125282-T0-Plasma | Non-cancer control | 12494728 | 0 | 0 |
| 125319-T0-Plasma | Non-cancer control | 13189557 | 0 | 0 |
| 126025-T0-Plasma | Non-cancer control | 12740549 | 0 | 0 |
| 126036-T0-Plasma | Non-cancer control | 14795994 | 0 | 0 |
| 220035-T0-Plasma | Non-cancer control | 13109701 | 0 | 0 |
| 220080-T0-Plasma | Non-cancer control | 15418317 | 0 | 0 |
| 220083-T0-Plasma | Non-cancer control | 15394113 | 0 | 0 |
| 220117-T0-Plasma | Non-cancer control | 14416278 | 0 | 0 |
| 220144-T0-Plasma | Non-cancer control | 13264082 | 0 | 0 |
| 220166-T0-Plasma | Non-cancer control | 11942131 | 0 | 0 |
| 220216-T0-Plasma | Non-cancer control | 13887202 | 0 | 0 |
| 220254-T0-Plasma | Non-cancer control | 15226033 | 0 | 0 |
| 220323-T0-Plasma | Non-cancer control | 13629173 | 0 | 0 |
| 220386-T0-Plasma | Non-cancer control | 14435615 | 0 | 0 |
| 220473-T0-Plasma | Non-cancer control | 13977308 | 0 | 0 |

To improve the sensitivity of ctHPV-DNA detection, a target-enrichment step was adopted using capture hybridization with probes specific for HPV DNA and only few human genes as described in Table 1. The sequencing libraries after target enrichment revealed detection of HPV-derived DNA in all 33 HPV-positive OPSCCs at baseline (Figure 2B): with 100% sensitivity a significant improvement over lcWGS (P = 0.013, McNemar's Chi-squared test). Absolute mapped HPV16 reads varied from 100 to over a million at baseline in the plasma of OPSCC patients with HPV16-positive tumors (Table 3).

**Table 3:**

| **Sample** | **Human reads** | **HPV reads** | **HPV:Human (x 1E+06)** | **Patient** | **Type** | **Timepoint** | **Group** | **HPV16 mean coverage** |
|---|---|---|---|---|---|---|---|---|
| 110192-M6-Plasma | 1099860 | 1451 | 1319.26 | 110192 | Plasma | M6 | Relapse | 33.10 |
| 110192-T0-Plasma | 1675671 | 4343 | 2591.80 | 110192 | Plasma | T0 | Relapse | 82.91 |
| 110299-M12-Plasma | 2032098 | 0 | 0 | 110299 | Plasma | M12 | Relapse | 0 |
| 110299-M24-Plasma | 1132161 | 0 | 0 | 110299 | Plasma | M24 | Relapse | 0 |
| 110299-M6-Plasma | 2783185 | 0 | 0 | 110299 | Plasma | M6 | Relapse | 0 |
| 110299-T0-Plasma | 1841832 | 191206 | 103812.94 | 110299 | Plasma | T0 | Relapse | 4066.85 |
| 110362-M6-Plasma | 726283 | 878 | 1208.90 | 110362 | Plasma | M6 | Relapse | 18.41 |
| 110362-T0-Plasma | 2034262 | 902 | 443.40 | 110362 | Plasma | T0 | Relapse | 19.86 |
| 115004-M12-Plasma | 895792 | 335 | 373.97 | 115004 | Plasma | M12 | Relapse | 6.09 |
| 115004-M24-Plasma | 1852668 | 0 | 0 | 115004 | Plasma | M24 | Relapse | 0 |
| 115004-M6-Plasma | 821673 | 388 | 472.21 | 115004 | Plasma | M6 | Relapse | 7.95 |
| 115004-T0-Plasma | 866379 | 3831 | 4421.85 | 115004 | Plasma | T0 | Relapse | 71.74 |
| 115057-M6-Plasma | 1514343 | 436 | 287.91 | 115057 | Plasma | M6 | Relapse | 8.21 |
| 115057-T0-Plasma | 916135 | 95433 | 104169.15 | 115057 | Plasma | T0 | Relapse | 1869.95 |
| 115116-M12-Plasma | 1398218 | 0 | 0 | 115116 | Plasma | M12 | Relapse | 0 |
| 115116-M24-Plasma | 1558027 | 400 | 256.73 | 115116 | Plasma | M24 | Relapse | 8.59 |
| 115116-M6-Plasma | 1481527 | 1 | 0.67 | 115116 | Plasma | M6 | Relapse | 0.02 |
| 115116-T0-Plasma | 1877418 | 100 | 53.26 | 115116 | Plasma | T0 | Relapse | 1.72 |
| 115200-M12-Plasma | 1939346 | 0 | 0 | 115200 | Plasma | M12 | Relapse | 0 |
| 115200-M24-Plasma | 663692 | 0 | 0 | 115200 | Plasma | M24 | Relapse | 1076.18 |
| 115200-T0-Plasma | 1948201 | 51601 | 26486.49 | 115200 | Plasma | T0 | Relapse | 0 |
| 115295-M12-Plasma | 1206919 | 0 | 0 | 115295 | Plasma | M12 | Relapse | 0 |
| 115295-M24-Plasma | 1054551 | 0 | 0 | 115295 | Plasma | M24 | Relapse | 0 |
| 115295-M6-Plasma | 470327 | 0 | 0 | 115295 | Plasma | M6 | Relapse | 38.38 |
| 115295-T0-Plasma | 2097684 | 2134 | 1017.31 | 115295 | Plasma | T0 | Relapse | 0 |
| 210117-M12-Plasma | 1927740 | 868 | 450.27 | 210117 | Plasma | M12 | Relapse | 18.47 |
| 210117-M24-Plasma | 1373599 | 288 | 209.67 | 210117 | Plasma | M24 | Relapse | 6.22 |
| 210117-M6-Plasma | 1037689 | 35 | 33.73 | 210117 | Plasma | M6 | Relapse | 0.81 |
| 210117-T0-Plasma | 2056075 | 11603 | 5643.28 | 210117 | Plasma | T0 | Relapse | 271.98 |
| 210193-M12-Plasma | 1365234 | 10253 | 7510.07 | 210193 | Plasma | M12 | Relapse | 190.37 |
| 210193-M24-Plasma | 1763578 | 29280 | 16602.61 | 210193 | Plasma | M24 | Relapse | 547.69 |
| 210193-M6-Plasma | 793603 | 180 | 226.81 | 210193 | Plasma | M6 | Relapse | 3.82 |
| 210193-T0-Plasma | 1933045 | 3723 | 1925.98 | 210193 | Plasma | T0 | Relapse | 83.40 |
| 210281-M12-Plasma | 1659825 | 0 | 0 | 210281 | Plasma | M12 | Relapse | 0 |
| 210281-M24-Plasma | 946884 | 4 | 4.22 | 210281 | Plasma | M24 | Relapse | 0.08 |
| 210281-M6-Plasma | 1080182 | 0 | 0 | 210281 | Plasma | M6 | Relapse | 0 |
| 210281-T0-Plasma | 2076294 | 5276 | 2541.07 | 210281 | Plasma | T0 | Relapse | 105.30 |
| 310337-M24-Plasma | 1677215 | 1234991 | 736334.34 | 310337 | Plasma | M24 | Relapse | 25048.20 |
| 310337-M6-Plasma | 1067249 | 156 | 146.17 | 310337 | Plasma | M6 | Relapse | 2.70 |
| 310337-T0-Plasma | 2285647 | 36241 | 15855.90 | 310337 | Plasma | T0 | Relapse | 730.02 |
| HND-21-014-T0-Plasma | 1114965 | 480 | 430.51 | HND-21-014 | Plasma | T0 | Relapse | 8.48 |
| HND-21-014-T1-Plasma | 675220 | 0 | 0 | HND-21-014 | Plasma | T1 | Relapse | 0 |
| HND-21-014-T2-Plasma | 2580076 | 0 | 0 | HND-21-014 | Plasma | T2 | Relapse | 0 |
| HND-21-014-T3-Plasma | 2617349 | 0 | 0 | HND-21-014 | Plasma | T3 | Relapse | 0 |
| HND-21-014-T4-Plasma | 1337264 | 0 | 0 | HND-21-014 | Plasma | T4 | Relapse | 0 |
| HND-21-014-T5-Plasma | 2067789 | 0 | 0 | HND-21-014 | Plasma | T5 | Relapse | 0 |
| HND-21-014-T6-Plasma | 1834458 | 0 | 0 | HND-21-014 | Plasma | T6 | Relapse | 0 |
| HND-21-029-T0-Plasma | 1063180 | 1671 | 1571.70 | HND-21-029 | Plasma | T0 | Relapse | 33.85 |
| HND-21-029-T1-Plasma | 1606438 | 0 | 0 | HND-21-029 | Plasma | T1 | Relapse | 0 |
| HND-21-029-T2-Plasma | 2733259 | 1 | 0.37 | HND-21-029 | Plasma | T2 | Relapse | 0.02 |
| HND-21-029-T3-Plasma | 630157 | 0 | 0 | HND-21-029 | Plasma | T3 | Relapse | 0 |
| HND-21-029-T4-Plasma | 1032343 | 2 | 1.94 | HND-21-029 | Plasma | T4 | Relapse | 0.03 |
| HND-21-029-T5-Plasma | 2092658 | 102 | 48.74 | HND-21-029 | Plasma | T5 | Relapse | 2.51 |
| HND-22-048-T0-Plasma | 1225432 | 26148 | 21337.78 | HND-22-048 | Plasma | T0 | Relapse | 490.98 |
| HND-22-048-T1-Plasma | 1349430 | 1 | 0.74 | HND-22-048 | Plasma | T1 | Relapse | 0.01 |
| HND-22-048-T2-Plasma | 1048835 | 0 | 0 | HND-22-048 | Plasma | T2 | Relapse | 0 |
| HND-22-048-T3-Plasma | 1424482 | 0 | 0 | HND-22-048 | Plasma | T3 | Relapse | 0 |
| 110187-M12-Plasma | 1524190 | 1 | 0.66 | 110187 | Plasma | M12 | Disease free follow-up | 0.02 |
| 110187-M24-Plasma | 1114676 | 1 | 0.90 | 110187 | Plasma | M24 | Disease free follow-up | 0.02 |
| 110187-M6-Plasma | 527449 | 0 | 0 | 110187 | Plasma | M6 | Disease free follow-up | 0 |
| 110187-T0-Plasma | 1965269 | 2772 | 1410.49 | 110187 | Plasma | T0 | Disease free follow-up | 64.29 |
| 110240-M12-Plasma | 1620704 | 0 | 0 | 110240 | Plasma | M12 | Disease free follow-up | 0 |
| 110240-M6-Plasma | 718996 | 0 | 0 | 110240 | Plasma | M6 | Disease free follow-up | 0 |
| 110240-T0-Plasma | 2034164 | 33211 | 16326.61 | 110240 | Plasma | T0 | Disease free follow-up | 659.05 |
| 110251-M12-Plasma | 1214089 | 0 | 0 | 110251 | Plasma | M12 | Disease free follow-up | 0 |
| 110251-M24-Plasma | 883467 | 7 | 7.92 | 110251 | Plasma | M24 | Disease free follow-up | 0.14 |
| 110251-M6-Plasma | 668709 | 0 | 0 | 110251 | Plasma | M6 | Disease free follow-up | 0 |
| 110251-T0-Plasma | 1696644 | 2798 | 1649.14 | 110251 | Plasma | T0 | Disease free follow-up | 57.68 |
| 110343-M12-Plasma | 1412370 | 0 | 0 | 110343 | Plasma | M12 | Disease free follow-up | 0 |
| 110343-M24-Plasma | 1179284 | 0 | 0 | 110343 | Plasma | M24 | Disease free follow-up | 0 |
| 110343-M6-Plasma | 1144310 | 0 | 0 | 110343 | Plasma | M6 | Disease free follow-up | 0 |
| 110343-T0-Plasma | 1511094 | 8357 | 5530.43 | 110343 | Plasma | T0 | Disease free follow-up | 163.64 |
| 110357-M12-Plasma | 1882819 | 0 | 0 | 110357 | Plasma | M12 | Disease free follow-up | 0 |
| 110357-M24-Plasma | 1258756 | 0 | 0 | 110357 | Plasma | M24 | Disease free follow-up | 0 |
| 110357-M6-Plasma | 1545236 | 0 | 0 | 110357 | Plasma | M6 | Disease free follow-up | 0 |
| 110357-T0-Plasma | 1939130 | 33746 | 17402.65 | 110357 | Plasma | T0 | Disease free follow-up | 793.99 |
| 110383-M12-Plasma | 1750740 | 0 | 0 | 110383 | Plasma | M12 | Disease free follow-up | 0 |
| 110383-M6-Plasma | 1327280 | 0 | 0 | 110383 | Plasma | M6 | Disease free follow-up | 0 |
| 110383-T0-Plasma | 2360974 | 2053 | 869.56 | 110383 | Plasma | T0 | Disease free follow-up | 44.88 |
| 115168-M12-Plasma | 2672745 | 0 | 0 | 115168 | Plasma | M12 | Disease free follow-up | 0 |
| 115168-M24-Plasma | 1293618 | 0 | 0 | 115168 | Plasma | M24 | Disease free follow-up | 0 |
| 115168-M6-Plasma | 1602191 | 0 | 0 | 115168 | Plasma | M6 | Disease free follow-up | 0.16 |
| 115168-T0-Plasma | 2070453 | 243 | 117.37 | 115168 | Plasma | T0 | Disease free follow-up | 0 |
| 115315-M12-Plasma | 1987789 | 0 | 0 | 115315 | Plasma | M12 | Disease free follow-up | 0 |
| 115315-M24-Plasma | 1377329 | 0 | 0 | 115315 | Plasma | M24 | Disease free follow-up | 0.04 |
| 115315-M6-Plasma | 992547 | 1 | 1.01 | 115315 | Plasma | M6 | Disease free follow-up | 0 |
| 115315-T0-Plasma | 1472604 | 738 | 501.15 | 115315 | Plasma | T0 | Disease free follow-up | 0 |
| 115376-M12-Plasma | 1299471 | 0 | 0 | 115376 | Plasma | M12 | Disease free follow-up | 0 |
| 115376-M6-Plasma | 1146492 | 1 | 0.87 | 115376 | Plasma | M6 | Disease free follow-up | 0.02 |
| 115376-T0-Plasma | 1712859 | 28148 | 16433.34 | 115376 | Plasma | T0 | Disease free follow-up | 526.76 |
| 116037-M6-Plasma | 1063089 | 0 | 0 | 116037 | Plasma | M6 | Disease free follow-up | 0 |
| 116037-T0-Plasma | 2106234 | 1087 | 516.09 | 116037 | Plasma | T0 | Disease free follow-up | 21.32 |
| 210005-M12-Plasma | 1189458 | 25 | 21.02 | 210005 | Plasma | M12 | Disease free follow-up | 0.69 |
| 210005-M24-Plasma | 1496829 | 0 | 0 | 210005 | Plasma | M24 | Disease free follow-up | 0 |
| 210005-M6-Plasma | 1509071 | 0 | 0 | 210005 | Plasma | M6 | Disease free follow-up | 0 |
| 210005-T0-Plasma | 1885986 | 6540 | 3467.68 | 210005 | Plasma | T0 | Disease free follow-up | 126.84 |
| 210199-M12-Plasma | 1602444 | 0 | 0 | 210199 | Plasma | M12 | Disease free follow-up | 0 |
| 210199-M24-Plasma | 1723452 | 5 | 2.90 | 210199 | Plasma | M24 | Disease free follow-up | 0.09 |
| 210199-M6-Plasma | 1361545 | 0 | 0 | 210199 | Plasma | M6 | Disease free follow-up | 0 |
| 210199-T0-Plasma | 1093729 | 1935 | 1769.18 | 210199 | Plasma | T0 | Disease free follow-up | 36.90 |
| 210200-M12-Plasma | 1369949 | 0 | 0 | 210200 | Plasma | M12 | Disease free follow-up | 0 |
| 210200-M24-Plasma | 1270060 | 0 | 0 | 210200 | Plasma | M24 | Disease free follow-up | 0 |
| 210200-M6-Plasma | 1484376 | 0 | 0 | 210200 | Plasma | M6 | Disease free follow-up | 0 |
| 210200-T0-Plasma | 1730218 | 676985 | 391271.50 | 210200 | Plasma | T0 | Disease free follow-up | 13817.99 |
| 210348-M12-Plasma | 1211729 | 0 | 0 | 210348 | Plasma | M12 | Disease free follow-up | 0 |
| 210348-M24-Plasma | 883798 | 0 | 0 | 210348 | Plasma | M24 | Disease free follow-up | 0 |
| 210348-M6-Plasma | 2175844 | 0 | 0 | 210348 | Plasma | M6 | Disease free follow-up | 0 |
| 210348-T0-Plasma | 1866669 | 1135053 | 608063.35 | 210348 | Plasma | T0 | Disease free follow-up | 24737.57 |
| 210457-M12-Plasma | 1216666 | 0 | 0 | 210457 | Plasma | M12 | Disease free follow-up | 0 |
| 210457-M24-Plasma | 1385020 | 1 | 0.72 | 210457 | Plasma | M24 | Disease free follow-up | 0.03 |
| 210457-M6-Plasma | 1517416 | 0 | 0 | 210457 | Plasma | M6 | Disease free follow-up | 0 |
| 210457-T0-Plasma | 1728733 | 906 | 524.08 | 210457 | Plasma | T0 | Disease free follow-up | 19.87 |
| 410511-M12-Plasma | 1197842 | 0 | 0 | 410511 | Plasma | M12 | Disease free follow-up | 0 |
| 410511-M6-Plasma | 1264900 | 0 | 0 | 410511 | Plasma | M6 | Disease free follow-up | 0 |
| 410511-T0-Plasma | 1912632 | 383322 | 200415.97 | 410511 | Plasma | T0 | Disease free follow-up | 8115.61 |
| HND-21-013-T0-Plasma | 1529208 | 18785 | 12284.14 | HND-21-013 | Plasma | T0 | Disease free follow-up | 332.10 |
| HND-21-013-T1-Plasma | 816435 | 0 | 0 | HND-21-013 | Plasma | T1 | Disease free follow-up | 0 |
| HND-21-013-T2-Plasma | 507701 | 0 | 0 | HND-21-013 | Plasma | T2 | Disease free follow-up | 0 |
| HND-21-013-T3-Plasma | 1407364 | 0 | 0 | HND-21-013 | Plasma | T3 | Disease free follow-up | 0 |
| HND-21-040-T0-Plasma | 2343868 | 1186 | 506.00 | HND-21-040 | Plasma | T0 | Disease free follow-up | 21.58 |
| HND-21-040-T1-Plasma | 3738735 | 0 | 0 | HND-21-040 | Plasma | T1 | Disease free follow-up | 0 |
| HND-21-040-T2-Plasma | 1193416 | 0 | 0 | HND-21-040 | Plasma | T2 | Disease free follow-up | 0 |
| HND-21-040-T3-Plasma | 1297217 | 0 | 0 | HND-21-040 | Plasma | T3 | Disease free follow-up | 0 |
| HND-21-040-T4-Plasma | 1192694 | 0 | 0 | HND-21-040 | Plasma | T4 | Disease free follow-up | 0 |
| HND-21-040-T5-Plasma | 1607521 | 0 | 0 | HND-21-040 | Plasma | T5 | Disease free follow-up | 0 |
| HND-21-040-T6-Plasma | 1807308 | 0 | 0 | HND-21-040 | Plasma | T6 | Disease free follow-up | 0 |
| 120121-T0-Plasma | 1123996 | 0 | 0 | 120121 | Plasma | T0 | Non-cancer control | 0 |
| 120186-T0-Plasma | 1704704 | 0 | 0 | 120186 | Plasma | T0 | Non-cancer control | 0 |
| 120220-T0-Plasma | 2050556 | 1 | 0.49 | 120220 | Plasma | T0 | Non-cancer control | 0.02 |
| 120229-T0-Plasma | 1869902 | 0 | 0 | 120229 | Plasma | T0 | Non-cancer control | 0 |
| 120265-T0-Plasma | 1212003 | 0 | 0 | 120265 | Plasma | T0 | Non-cancer control | 0 |
| 120272-T0-Plasma | 2068076 | 0 | 0 | 120272 | Plasma | T0 | Non-cancer control | 0 |
| 125197-T0-Plasma | 2170832 | 0 | 0 | 125197 | Plasma | T0 | Non-cancer control | 0 |
| 125245-T0-Plasma | 2245084 | 0 | 0 | 125245 | Plasma | T0 | Non-cancer control | 0 |
| 125282-T0-Plasma | 2013770 | 0 | 0 | 125282 | Plasma | T0 | Non-cancer control | 0 |
| 125319-T0-Plasma | 1042946 | 0 | 0 | 125319 | Plasma | T0 | Non-cancer control | 0 |
| 126025-T0-Plasma | 1441361 | 0 | 0 | 126025 | Plasma | T0 | Non-cancer control | 0 |
| 126036-T0-Plasma | 1884097 | 0 | 0 | 126036 | Plasma | T0 | Non-cancer control | 0 |
| 220035-T0-Plasma | 2012990 | 0 | 0 | 220035 | Plasma | T0 | Non-cancer control | 0 |
| 220080-T0-Plasma | 2039623 | 1 | 0.49 | 220080 | Plasma | T0 | Non-cancer control | 0 |
| 220083-T0-Plasma | 1923810 | 0 | 0 | 220083 | Plasma | T0 | Non-cancer control | 0 |
| 220117-T0-Plasma | 1833069 | 0 | 0 | 220117 | Plasma | T0 | Non-cancer control | 0 |
| 220144-T0-Plasma | 1934586 | 0 | 0 | 220144 | Plasma | T0 | Non-cancer control | 0 |
| 220166-T0-Plasma | 1755235 | 0 | 0 | 220166 | Plasma | T0 | Non-cancer control | 0 |
| 220200-T0-Plasma | 1195016 | 0 | 0 | 220200 | Plasma | T0 | Non-cancer control | 0 |
| 220216-T0-Plasma | 1962760 | 0 | 0 | 220216 | Plasma | T0 | Non-cancer control | 0 |
| 220254-T0-Plasma | 2410904 | 0 | 0 | 220254 | Plasma | T0 | Non-cancer control | 0 |
| 220323-T0-Plasma | 2242262 | 0 | 0 | 220323 | Plasma | T0 | Non-cancer control | 0 |
| 220386-T0-Plasma | 2169836 | 0 | 0 | 220386 | Plasma | T0 | Non-cancer control | 0 |
| 220473-T0-Plasma | 1652178 | 0 | 0 | 220473 | Plasma | T0 | Non-cancer control | 0 |
| 320105-T0-Plasma | 943463 | 0 | 0 | 320105 | Plasma | T0 | Non-cancer control | 0 |
| 320118-T0-Plasma | 814331 | 0 | 0 | 320118 | Plasma | T0 | Non-cancer control | 0 |
| 320270-T0-Plasma | 641275 | 0 | 0 | 320270 | Plasma | T0 | Non-cancer control | 0 |
| 420340-T0-Plasma | 1040183 | 0 | 0 | 420340 | Plasma | T0 | Non-cancer control | 0 |
| 420511-T0-Plasma | 835392 | 0 | 0 | 420511 | Plasma | T0 | Non-cancer control | 0 |
| 420587-T0-Plasma | 960890 | 0 | 0 | 420587 | Plasma | T0 | Non-cancer control | 0 |

For the lcWGS assay the number of HPV reads per million human genome reads was calculated and the cut-off was set at about 0.125 HPV reads per million human genome reads. Due to the enrichment of HPV in the next part of the experiment, the cut-off for positivity needed to be redefined, and the human genome reads were decided to be used for that along with routine sequencing 29 head and neck cancer genes as well. Upon observing the normal distribution of log-transformed read ratios in HPV-positive patients, a threshold of the mean log-transformed HPV per human genome read ratio minus three times the standard deviation was decided upon. This yielded a cut-off read ratio of about 4.73 HPV reads per million human genome reads.

In non-cancer controls two samples with one HPV16 read were observed and all others had no HPV reads at all, highlighting the perfect separation between HPV16-positive cases and non-cancer controls.

Given the predominant result of 0 HPV reads in controls, the controls were marginally informative for determining a cut-off above which a sample should be considered HPV-positive. As an alternative =the mean of the log-transformed HPV mapped reads of all 33 HPV16-positive samples minus 3x the standard deviation was determined as clinical cut-off, which statistically encompasses more than 99.9 % of positive samples. This clinical cut-off value was 4.73 HPV reads per million human genome reads. Values above the cut-off indicate a positive sample and equal to or below the cut-off a negative sample.

An absolute minimum number of HPV reads was not applied. In theory one HPV read per 10,000 human genome reads would be above the cut-off and indicate a positive test. However, one HPV read would not give much confidence and 3 or 5 reads seems more reasonable. The minimal number of reads observed at baseline was 100 with almost 2 million human genome reads sequenced.

Finally, the calculated cut-off was used to estimate the number of human genome reads required to call a sample with certain confidence negative. The cut-off of 4.73 per million means one read per 211 416 human genome reads. For a 95% confidence level 414 375 and for a 90% confidence level 346 722 human genome reads should be sequenced. Hence, above 500,000 human genome reads and no HPV reads, a sample can be called negative with confidence.

Within the studied cohort 100% sensitivity (95% confidence interval: 0.86 - 1) was observed when using target enrichment sequencing, in line with the expected ~99.9% based on the cut-off that was set on basis of 3x the standard deviation. None of the 30 non-cancer control samples reached the cut-off for positivity, in fact most had no reads at all, giving the test a specificity of 100% for detecting HPV-positive samples (95% confidence interval: 0.87 - 1).

Several lines of evidence reported previously indicate that cell-free DNA fragments of tumor cells are smaller than those derived from genomic DNA. This aspect was analyzed by comparing HPV DNA fragment lengths with human DNA fragment lengths. Figure 1C demonstrates the different size distributions of HPV and human DNA fragments in the plasma; the median HPV DNA fragment length was 147 bp and that of human DNA 172 bp (P = 3.0 * 10-14). The fragment sizes of HPV DNA were found to be in line with those reported from tumor cells.

### Example 2: ctHPV-DNA in oral rinses at baseline

In order to explore whether analysis of oral rinses may improve ctHPV-DNA detection, target enrichment sequencing was performed on oral rinse samples of the same patient and non-cancer control group at baseline. Data is provided in Table 4.

**Table 4:**

| **Sample** | **Human reads** | **HPV reads** | **HPV:Human (x 1E+06)** | **Patient** | **Type** | **Timepoint** | **Group** | **HPV16 mean coverage** |
|---|---|---|---|---|---|---|---|---|
| 110192-T0-OR | 1721534 | 40 | 23.24 | 110192 | OR | T0 | Relapse | 1.06 |
| 110299-T0-OR | 1372773 | 31443 | 22904.73 | 110299 | OR | T0 | Relapse | 724.24 |
| 110362-T0-OR | 1041106 | 0 | 0 | 110362 | OR | T0 | Relapse | 0 |
| 115116-T0-OR | 1127303 | 0 | 0 | 115116 | OR | T0 | Relapse | 0 |
| 210117-T0-OR | 1507296 | 0 | 0 | 210117 | OR | T0 | Relapse | 0 |
| 210193-T0-OR | 1644348 | 0 | 0 | 210193 | OR | T0 | Relapse | 0 |
| 210281-T0-OR | 1156696 | 1133 | 979.51 | 210281 | OR | T0 | Relapse | 25.47 |
| 310337-T0-OR | 1711758 | 8 | 4.67 | 310337 | OR | T0 | Relapse | 0.17 |
| 110187-T0-OR | 2564922 | 1942 | 757.14 | 110187 | OR | T0 | Disease free follow-up | 43.53 |
| 110240-T0-OR | 1045037 | 116735 | 111704.18 | 110240 | OR | T0 | Disease free follow-up | 2192.39 |
| 110251-T0-OR | 829840 | 69 | 83.15 | 110251 | OR | T0 | Disease free follow-up | 1.62 |
| 110343-T0-OR | 1868711 | 1409 | 754.00 | 110343 | OR | T0 | Disease free follow-up | 32.50 |
| 110357-T0-OR | 914247 | 182 | 199.07 | 110357 | OR | T0 | Disease free follow-up | 3.67 |
| 110383-T0-OR | 1590686 | 8903 | 5596.96 | 110383 | OR | T0 | Disease free follow-up | 240.01 |
| 115168-T0-OR | 1001439 | 8 | 7.99 | 115168 | OR | T0 | Disease free follow-up | 5.41 |
| 115315-T0-OR | 1801521 | 0 | 0 | 115315 | OR | T0 | Disease free follow-up | 14.59 |
| 115376-T0-OR | 1207053 | 0 | 0 | 115376 | OR | T0 | Disease free follow-up | 0 |
| 116037-T0-OR | 1484170 | 0 | 0 | 116037 | OR | T0 | Disease free follow-up | 0 |
| 210005-T0-OR | 2013330 | 423 | 210.10 | 210005 | OR | T0 | Disease free follow-up | 11.17 |
| 210200-T0-OR | 1044025 | 1 | 0.96 | 210200 | OR | T0 | Disease free follow-up | 0.02 |
| 210348-T0-OR | 916379 | 1 | 1.09 | 210348 | OR | T0 | Disease free follow-up | 0.04 |
| 210457-T0-OR | 1848762 | 0 | 0 | 210457 | OR | T0 | Disease free follow-up | 0 |
| 410511-T0-OR | 1089322 | 4893 | 4491.78 | 410511 | OR | T0 | Disease free follow-up | 104.76 |
| 120186-T0-OR | 1705339 | 0 | 0 | 120186 | OR | T0 | Non-cancer control | 0 |
| 120229-T0-OR | 1241430 | 0 | 0 | 120229 | OR | T0 | Non-cancer control | 0 |
| 120265-T0-OR | 1341736 | 18 | 13.42 | 120265 | OR | T0 | Non-cancer control | 0.38 |
| 120272-T0-OR | 1599121 | 0 | 0 | 120272 | OR | T0 | Non-cancer control | 0 |
| 125197-T0-OR | 1347319 | 0 | 0 | 125197 | OR | T0 | Non-cancer control | 0 |
| 125245-T0-OR | 1138803 | 0 | 0 | 125245 | OR | T0 | Non-cancer control | 0 |
| 125282-T0-OR | 1381051 | 0 | 0 | 125282 | OR | T0 | Non-cancer control | 0 |
| 125319-T0-OR | 1135694 | 0 | 0 | 125319 | OR | T0 | Non-cancer control | 0 |
| 126025-T0-OR | 1133213 | 0 | 0 | 126025 | OR | T0 | Non-cancer control | 0 |
| 126036-T0-OR | 1630745 | 0 | 0 | 126036 | OR | T0 | Non-cancer control | 0 |
| 220035-T0-OR | 2534790 | 0 | 0 | 220035 | OR | T0 | Non-cancer control | 0 |
| 220080-T0-OR | 1096417 | 0 | 0 | 220080 | OR | T0 | Non-cancer control | 0 |
| 220083-T0-OR | 1304687 | 0 | 0 | 220083 | OR | T0 | Non-cancer control | 0 |
| 220117-T0-OR | 8127354 | 0 | 0 | 220117 | OR | T0 | Non-cancer control | 0 |
| 220144-T0-OR | 1596469 | 0 | 0 | 220144 | OR | T0 | Non-cancer control | 0 |
| 220166-T0-OR | 1279271 | 0 | 0 | 220166 | OR | T0 | Non-cancer control | 0 |
| 220216-T0-OR | 1997493 | 0 | 0 | 220216 | OR | T0 | Non-cancer control | 0 |
| 220254-T0-OR | 1918255 | 0 | 0 | 220254 | OR | T0 | Non-cancer control | 0 |
| 220323-T0-OR | 963076 | 0 | 0 | 220323 | OR | T0 | Non-cancer control | 0 |
| 220386-T0-OR | 1889450 | 0 | 0 | 220386 | OR | T0 | Non-cancer control | 0 |
| 220473-T0-OR | 1678729 | 0 | 0 | 220473 | OR | T0 | Non-cancer control | 0 |
| 320105-T0-OR | 1772675 | 6 | 3.38 | 320105 | OR | T0 | Non-cancer control | 0 |
| 320118-T0-OR | 1819024 | 1 | 0.55 | 320118 | OR | T0 | Non-cancer control | 0.01 |
| 320270-T0-OR | 2501920 | 1 | 0.40 | 320270 | OR | T0 | Non-cancer control | 0.02 |
| 420340-T0-OR | 2316574 | 0 | 0 | 420340 | OR | T0 | Non-cancer control | 0 |

ctHPV-DNA was detected at baseline in 17 of 33 oral rinse samples of patients with an HPV-positive tumor. In the group of non-cancer controls it was found that two oral rinses produced 6 and 18 HPV reads respectively. Oral rinse samples thus proved less sensitive and less specific for the detection of ctHPV-DNA. Due to the extremely favorable results of the plasma samples over oral rinses it was decided not to continue with the oral rinses in the longitudinal follow-up study.

### Example 3: Recurrent disease detection by longitudinal ctHPV-DNA analysis

To demonstrate the effectiveness of longitudinal monitoring of ctHPV-DNA in plasma for accurately predicting residual disease or disease recurrence during post-treatment follow-up, plasma samples of all available time points were initially analyzed for ctHPV-DNA positivity according to the abovementioned cut-off. Figure 3 shows representative follow-up plots of two cases who remained disease-free (Figure 3A and B). In the 18 disease-free cases analyzed, all 76 samples remained below the threshold except for one single observation; one patient who remained disease-free showed at twelve months one plasma sample just above the background, before it turned to 0 at 24 months.

The plots in Figures 3C and D demonstrate typical examples of cases developing recurrence. CtHPV-DNA levels go down in all but one case, although in most cases (7 of 9) ctHPV-DNA remain present above background levels at the earliest time point after treatment, while there were no clinical indications of tumor residue. As an example, a particular patient (115116) presented with a TxN2a OPSCC in the right neck and was treated by a neck dissection right and adjuvant radiotherapy including elective treatment on the contralateral neck and the tonsillar region. In the plasma of this patient, elevated levels of ctHPV-DNA were found at about 6 months although not reaching the cut-off, which dropped to zero at about 12 months and rose at about 24 months. After about 27 months a regional recurrence was detected in the left side of the neck, which was treated with a neck dissection and post-operative radiotherapy.

All other cases developing recurrent disease also displayed detectable ctHPV-DNA in the plasma during follow-up and the respective plots are shown in Figure 5. Hence, above-threshold levels of ctHPV-DNA were detected in the plasma of all 9 recurrent cases. In 7 of 9 cases, ctHPV-DNA was detected in the plasma before imaging or clinical diagnosis of recurrent disease (Figure 3 C, D and Figure 5). In the other cases, the elevated ctHPV-DNA was detected approximately at the same time as diagnosis of recurrent disease.

### Example 4: Residual disease monitoring by longitudinal ctHPV-DNA analysis

In the studied patient group, six cases were diagnosed with residual disease in the neck. The cytology and physical examination of one patient (HND22-48) was suspect for residual disease, but it was decided to follow a watchful surveillance before neck dissection, and the patient remained disease free in the follow-up, in line with the ctHPV-DNA sequencing in plasma. Cytology was not conclusive in 5 patients and these underwent neck dissection. In four cases the pathologist reported a non-vital necrotic lymph node metastasis in the neck (Figures 4A, 5). Such non-vital and necrotic metastases can be cleared and have no clinical consequences for the patient when left *in situ.* The absence of ctHPV-DNA in the blood was in retrospect predictive of the non-vital status of the residual disease or the disease-free status in all five cases, underpinning the remarkable accuracy of ctHPV-DNA disease monitoring. In the remaining case (210281), a delayed lymph node metastasis was detected and treated before the first blood withdrawal (Figure 4B).

In Figure 4C, the disease-free survival is depicted of HPV-positive OPSCC patients with and without ctHPV-DNA in plasma during follow-up. The hazard of ctHPV-DNA positive patients was significantly higher (hazard ratio was infinite due to a lack of events in ctHPV-DNA negative patients).

Additional embodiments and features of the present disclosure will be apparent to one of ordinary skill in art based on the description provided herein. The embodiments herein provide various features and advantageous details thereof in the description. Descriptions of well-known/conventional methods and techniques are omitted so as to not unnecessarily obscure the embodiments herein.

The foregoing description fully reveals the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments in this disclosure have been described in terms of preferred embodiments, those skilled in the art will recognize that the embodiments herein can be practiced with modification within the spirit and scope of the embodiments as described herein, without departing from the principles of the disclosure.

Any discussion of documents, acts, materials, devices, articles and the like that has been included in this specification is solely for the purpose of providing a context for the disclosure. It is not to be taken as an admission that any or all of these matters form a part of the prior art base or were common general knowledge in the field relevant to the disclosure as it existed anywhere before the priority date of this application.

## Claims

1. A method for detecting and/or measuring expression of head and neck cancer marker(s) in a biological sample comprising:
a) isolating DNA from the biological sample; and
b) performing target-enrichment sequencing analysis on the isolated DNA for confirming presence or absence of marker(s) selected from a group comprising whole genome of HPV16, E7 sequence(s) of one or more of HPV18, HPV31, HPV33, HPV35, HPV39, HPV45, HPV51, HPV52, HPV56, HPV58, HPV59, HPV68, HPV73 and HPV82, and combination(s) thereof.

2. The method of claim 1, wherein the marker(s) may further comprise one or more human genes frequently mutated in head and neck cancer.

3. The method of claim 2, wherein the human genes are selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63 or combinations thereof.

4. The method of any of claims 1-3 **characterized by** at least about 90% specificity and at least about 90% selectivity.

5. The method of any of claims 1-4, wherein the method further comprises aligning reads of the HPV and/or human marker(s) to a human genome reference sequence.

6. A method of diagnosing head and neck cancer in a subject, comprising:
c) Isolating DNA from a biological sample obtained from a subject; and
d) Performing target-enrichment sequencing analysis on the isolated DNA for confirming presence or absence of marker(s) selected from a group comprising
∘ whole genome of HPV16,
∘ E7 sequence(s) of one or more of HPV18, HPV31, HPV33, HPV35, HPV39, HPV45, HPV51, HPV52, HPV56, HPV58, HPV59, HPV68, HPV73 and HPV82,
∘ human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
and combination(s) thereof.

7. The method as claimed in claim 6, wherein the head and neck cancer is recurrent head and neck cancer; and/or wherein the subject is one that has previously received treatment for and/or has previously recovered from head and neck cancer.

8. The method of claim 6, wherein about 3 to about 5 HPV reads per million human genome reads in the target-enrichment sequencing analysis is determined as a cut-off limit for confirming diagnosis of head and neck cancer in the subject.

9. A biomarker enrichment panel comprising probes specific for capture hybridization of biomaker(s) selected from a group comprising
o whole genome of HPV16,
o E7 sequence(s) of one or more of HPV18, HPV31, HPV33, HPV35, HPV39, HPV45, HPV51, HPV52, HPV56, HPV58, HPV59, HPV68, HPV73 and HPV82,
o human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
and combination(s) thereof for detection of head and neck cancer(s).

10. The biomarker enrichment panel of claim 9, wherein the Human:HPV enrichment probes are pooled at a ratio of about 2:1.

11. Use of maker(s) selected from a group comprising
- whole genome of HPV16,
- E7 sequence(s) of one or more of HPV18, HPV31, HPV33, HPV35, HPV39, HPV45, HPV51, HPV52, HPV56, HPV58, HPV59, HPV68, HPV73 and HPV82,
- human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
and combination(s) thereof for detection of head and neck cancer(s).

12. The use of claim 11, wherein the head and neck cancer is a recurrent head and neck cancer.

13. Use of target enrichment analysis for detecting and/or measuring expression of head and neck cancer marker(s) selected from a group comprising
o whole genome of HPV16,
o E7 sequence(s) of one or more of HPV18, HPV31, HPV33, HPV35, HPV39, HPV45, HPV51, HPV52, HPV56, HPV58, HPV59, HPV68, HPV73 and HPV82,
o human gene(s) selected from a group comprising AJUBA, ASXL1, CASP8, CDKN2A, CUL3, DDX3X, EPHA2, FAT1, FBXW7, FGFR3, HRAS, KDM6A, KEAP1, KMT2D, KRAS, NFE2L2, NOTCH1, NOTCH2, NSD1, PIK3CA, PTEN, RB1, RHOA, TERT promotor, TGFBR2, TP53, TP53 5'UTR and TP63,
and combination(s) thereof
in a biological sample, for detection of head and neck cancer(s).
